# EUROPEAN PATENT APPLICATION

(11) **EP 1 514 538 A1**
(43) Date of publication of application: **16.03.2005**
(21) Application number: 03736082.3
(22) Date of filing: 09.06.2003
(51) Int. Cl.: A61K 9/06, A61K 9/08, A61K 47/02, A61K 47/36, A61K 47/42

(54) **SUSTAINED-RELEASE COMPOSITION, PROCESS FOR PRODUCING THE SAME AND PREPARATION THEREOF**

(30) Priority: 20.06.2002 JP 2002179788; 25.12.2002 JP 2002374173
(71) Applicant: LTT Bio-Pharma Co., Ltd., Tokyo 105-6201 (JP)
(72) Inventor: MIZUSHIMA, Yutaka, Minato-ku, Tokyo 106-0032 (JP); TAKAGI, Yukie, Tama-ku, Kawasaki-shi, Kanagawa 214-0035 (JP); HAGI, Tomomi, Kanazawa-ku, Yokohama-shi, Kanagawa 236- (JP); IKOMA, Toshiyuki, Tsukuba-shi, Ibaraki 305-0047 (JP)
(74) Representative: Rupp, Christian, Dipl.Phys.
(86) International application number: PCT/JP2003/007251
(87) International publication number: WO 2004/000270

(57) **Abstract**

The present invention provides a sustained-release composition by which a sustained-release effect can be obtained for a long time when injecting microparticles of the composition in an amount that can be subcutaneously or intramuscularly injected to a human with ease and without pain. The composition comprises porous hydroxyapatite microparticles having pores embolized by filling the pores in the microparticles with a biologically active drug, a human serum protein, and a mucopolysaccharide, and adding a divalent metal ion. Alternatively, the composition comprises porous hydroxyapatite microparticles having pores embolized in the outer layer by filling the pores in the microparticles with a biologically active drug, a human serum protein, and a water-soluble calcium salt one after another or at one time, and then adding sodium carbonate, sodium hydrogen carbonate, or an aqueous carbonate ion solution.

## Description

### BACKGROUND OF THE INVENTION

### Field of the Invention

The present invention relates to a sustained-release composition comprising porous hydroxyapatite microparticles, a process for producing the same, and a preparation of the same. More particularly, the present invention relates to a sustained-release composition comprising embolized hydroxyapatite microparticles, a process for producing the same, a preparation of the same, and a sustained-release composition for skin.

### Description of the Related Art

Sustained-release preparations for injection can be applied to regenerative medicine, and have become further important in recent years. Until now, sustained-release preparations as inorganic or organic microparticles, capsules, hydrogels, or the like have been developed. Many injectable solutions exhibiting sustained release of a water-soluble drug for a long time using poly(lactic-co-glycolic acid) (PLGA) as a base have been studied (Japanese Patent Laid-Open No. 11-286403, Japanese Patent Laid-Open No. 2000-239104, Japanese Patent Laid-Open No. 2002-326960). Further, sustained-release microcapsules using PLGA containing a human growth hormone (hGH) as a base have been reported (Nature Medicine, 2: 795-799, 1996). In addition, sustained-release microcapsules using, as a base, PLGA containing leuprorelin as an LHRH agonist have been reported (Chemical Pharmaceutical Bulletin, 36: 1095-1103, 1988). PLGA is an *in vivo* digestive base that disappears by *in vivo* hydrolysis, and has properties preferable as a base for an injectable solution. when a sustained-release preparation using PLGA is produced, an organic solvent in which PLGA can be dissolved is generally used. However, hGH is denatured in an organic solvent, and a part of hGH is deactivated. Such a decreased activity not only impairs efficacy, but also involves the risk of an adverse effect on the living body. Since hGH is highly water-soluble, it is inevitable that an excessive amount of hGH is released in the early period of administration when a PLGA preparation is used. Further, use of a hydrogel or the like has been reported. However, it is usually difficult to administer a hydrogel by injection. Specifically, a thick needle by which a gel can be injected must be used, which is not favored by a patient. In addition, sustained-release particles using hydroxyapatite and a human growth hormone as a biologically active drug have been already reported (H. Gautier et al., Journal of Biomedical Material Research, 40, 606-613, 1998; J. Guicheux et al., Journal of Biomedical Material Research, 34, 165-170, 1997). However, these particles are two-component particles, the apatite has a large particle size of 40 to 80 µm or 200 µm which makes it difficult for the particles to be injected, and the *in vivo* sustained-release effect is unclear. The amount of hGH adsorbed on the apatite particles (entrapped amount) is small, specifically, 1% or less.

Furthermore, these sustained-release preparations have a problem in that the preparations burst, are organized to have considerably weak bioavailability, are not completely decomposed *in vivo,* or are not expected to exhibit long sustained release.

In order to solve these problems, the present inventors have attempted to produce a sustained-release preparation with nanospaces in porous hydroxyapatite microparticles embolized. As a result of studies up to the present, the inventors have found first of all that, due to the weak bioreactivity, hydroxyapatite is not organized, completely subcutaneously digested within two to five weeks depending on the manner of sintering, exhibits good bioavailability, does not burst, and has a considerable sustained-release effect when embolized.

Accordingly, an object of the present invention is to provide a sustained-release composition by which a sustained-release effect can be obtained for a long time when injecting microparticles of the composition in an amount that can be subcutaneously or intramuscularly injected to a human with ease and without pain, a process for producing the same, a preparation of the same, and a sustained-release composition for skin.

### SUMMARY OF THE INVENTION

In order to achieve the above object, the present invention provides a sustained-release composition comprising porous hydroxyapatite microparticles having pores charged by a biologically active drug (high-molecular-weight or low-molecular-weight drug), a human serum protein, or mucopolysaccharide, and then embolized by adding a divalent metal ion.

The present invention also provides a sustained-release composition comprising porous hydroxyapatite microparticles produced by which (1) the pores are charged by a biologically active drug, a human serum protein, and mucopolysaccharide, (2) the microparticles are freeze-dried fully or moderately, and (3) the resulting microparticles are embolized by a divalent metal ion solution.

The present invention further provides a sustained-release composition comprising porous hydroxyapatite microparticles having pores charged by a biologically active drug and a human serum protein, and then embolized in the outer layer by a divalent metal ion.

The present invention still further provides a sustained-release composition comprising porous hydroxyapatite microparticles having pores charged by a biologically active drug, human serum albumin, and water-soluble calcium salt one after another or at one time, and then embolized in the outer layer by adding sodium carbonate, sodium hydrogen carbonate, or an aqueous carbonate ion solution.

The present invention yet further provides a sustained-release composition comprising porous hydroxyapatite microparticles produced by which (1) the pores are charged by a biologically active drug, human serum albumin, and water-soluble calcium salt one after another or at one time, (2) the microparticles are freeze-dried fully or moderately, and then (3) the resulting microparticles are embolized by adding sodium carbonate, sodium hydrogen carbonate, or an aqueous carbonate ion solution.

The present invention further provides a sustained-release composition comprising porous hydroxyapatite microparticles having pores charged by a biologically active drug and water-soluble calcium salt one after another or at one time, and then embolized in the outer layer by adding sodium carbonate, sodium hydrogen carbonate, or an aqueous carbonate ion solution.

The present invention still further provides a sustained-release composition comprising porous hydroxyapatite microparticles having a biologically active drug highly binding to hydroxyapatite on the inner surface of their pores.

The present invention yet further provides a sustained-release composition comprising porous hydroxyapatite microparticles having pores binding the inner surface of pores in the microparticles with a biologically active drug, and further embolized by adding a divalent metal ion.

The present invention still further provides a sustained-release composition comprising porous hydroxyapatite microparticles having pores embolized by binding the inner surface of pores in the microparticles with a divalent metal ion, and further adding a biologically active drug.

The present invention provides a sustained-release composition for skin comprising porous hydroxyapatite microparticles filled with a dermatological therapeutic drug or a quasi-drug such as a sun screen together with a base, wherein the microparticles are mixed with an ointment, cream, or lotion.

In this sustained-release composition for skin, an appropriate amount of such a drug can be applied to the skin, because the drug is loaded into porous hydroxyapatite microparticles. Accordingly, the active ingredient is gradually released from the porous hydroxyapatite microparticles, and a dermatological therapeutic drug or a quasi-drug UV-absorbing substance such as a sun screen with which the porous hydroxyapatite microparticles are filled is gradually exuded(specifically, released slowly), whereby the effect of the composition lasts.

The present invention provides a process for producing a sustained-release composition, comprising mixing porous hydroxyapatite microparticles with an aqueous solution comprising a biologically active drug and a human serum protein and stirring the mixture to prepare a suspension, mixing the suspension with an aqueous mucopolysaccharide solution and a divalent metal ion solution, and separating a resulting solid from the mixture.

The present invention also provides a process for producing a sustained-release composition, comprising mixing porous hydroxyapatite microparticles with an aqueous solution comprising a biologically active drug and a human serum protein and stirring the mixture to prepare a suspension, mixing the suspension with an aqueous mucopolysaccharide solution and a divalent metal ion solution, separating a resulting solid from the mixture, and further freeze-drying the solid.

The present invention further provides a process for producing a sustained-release composition, comprising mixing porous hydroxyapatitemicroparticles with an aqueous solution comprising a biologically active drug and a human serum protein and stirring the mixture to prepare a suspension, mixing the suspension with an aqueous mucopolysaccharide solution, separating a resulting solid from the mixture, freeze-drying the solid, adding a divalent metal ion solution to the solid, and further freeze-drying the mixture.

The present invention still further provides a process for producing a sustained-release composition, comprising mixing porous hydroxyapatite microparticles with an aqueous solution comprising a biologically active drug, a human serum protein, and a water-soluble calcium salt and stirring the mixture to prepare a suspension, mixing the suspension with sodium carbonate, sodium hydrogen carbonate, or an aqueous carbonate ion solution, and separating a resulting solid from the mixture.

The present invention yet further provides a process for producing a sustained-release composition, comprising mixing porous hydroxyapatite microparticles with an aqueous solution comprising a biologically active drug, a human serum protein, and a water-soluble calcium salt and stirring the mixture to prepare a suspension, separating a resulting solid from the suspension, freeze-drying the solid, adding sodium carbonate, sodium hydrogen carbonate, or an aqueous carbonate ion solution to the solid, and further freeze-drying the mixture.

The present invention further provides a process for producing a sustained-release composition, comprising mixing porous hydroxyapatite microparticles with an aqueous solution comprising a biologically active drug and a water-soluble calcium salt and stirring the mixture to prepare a suspension, separating a resulting solid from the suspension, freeze-drying the solid, adding sodium carbonate, sodium hydrogen carbonate, or an aqueous carbonate ion solution to the solid, and further freeze-drying the mixture.

The present invention still further provides a process for producing a sustained-release composition, comprising mixing porous hydroxyapatite microparticles with an aqueous solution comprising a biologically active drug and stirring the mixture to prepare a suspension, mixing the suspension with a divalent metal ion solution, and separating a resulting solid from the mixture.

The present invention yet further provides a process for producing a sustained-release composition, comprising mixing porous hydroxyapatite microparticles with a divalent metal ion solution and stirring the mixture to prepare a suspension, mixing the suspension with an aqueous solution comprising a biologically active drug, and separating a resulting solid from the mixture.

The porous hydroxyapatite microparticles are preferably obtained by spray-drying a hydroxyapatite suspension and sintering the suspension at 100 to 800°C. This is because, if the sintering temperature is 800°C or more, the pores are crushed, and, if the temperature is 100°c or less, the suspension cannot be sintered.

The porous hydroxyapatite microparticles preferably have a pore diameter of 0.1 to 20 µm.

The biologically active drug is contained in the sustained-release composition preferably in an amount of at least 0.01 wt%.

The human serum protein is preferably human serum albumin or γ-globulin.

The human serum protein is preferably contained in the sustained-release composition in an amount of at least 1 wt%.

The divalent metal ion is preferably a zinc ion, copper ion, calcium ion, or magnesium ion.

The divalent metal ion is preferably contained in the sustained-release composition in an amount of at least 0.01 wt%. The mucopolysaccharide is preferably at least one of chondroitin sulfate, hyaluronic acid, heparin, heparan sulfate, dermatan sulfate, keratan sulfate, and these salts.

The mucopolysaccharide is preferably contained in the sustained-release composition in an amount of 1/100 or more of the human serum protein.

The sustained-release composition is preferably in a form suitable for subcutaneous injection, intradermal injection, intramuscular injection, intraocular administration, dermal application, and the like.

The sustained-release preparation of the present invention preferably comprises the composition into which a pharmaceutically acceptable additive is optionally incorporated.

The pharmaceutically acceptable additive is preferably a surfactant, preservative, or stabilizer.

The preparation is preferably freeze-dried.

The preparation is preferably in a form suitable for subcutaneous injection, intradermal injection, intramuscular injection, intraocular administration, dermal application, and the like.

The water-soluble calcium salt is preferably calcium chloride, calcium acetate, or calcium nitrate.

Features of the present invention will be further described below.

The embolizing of pores according to the present invention includes outer embolizing and entire embolizing. In each embolizing method, a drug is first incorporated in pores in porous hydroxyapatite singly or in a combination with a embolizing agent or a stabilizer, and then a precipitating agent, specifically, a divalent metal ion, sodium carbonate, sodium hydrogen carbonate, or an aqueous carbonate ion solution is added to precipitate the composition to form embolizations. Alternatively, a divalent metal ion is introduced in pores in porous hydroxyapatite, and then an aqueous solution of a biologically active drug is added; or an aqueous solution of a biologically active drug is incorporated in pores in porous hydroxyapatite, and then a divalent metal ion solution is added, to form a embolization. When embolizing only the outer layer, the pore interior is filled with the composition, and then a precipitating agent is added. For entire embolizing, the microparticles in which the composition is incorporated are once freeze-dried, air is let in the pores, and the precipitant is penetrated inside the microparticles, whereby embolizing can take place entirely.

In addition, (a) the size of porous hydroxyapatite microparticles, the size and volume of voids, and the suitable sintering temperature; (b) adoption or non-adoption of addition of only zinc salt or sodium carbonate in the last place, and preference for filling with a drug, a protein, and mucopolysaccharide as a mixed solution or sequential addition of a drug, a protein, and mucopolysaccharide, in the case of outer embolizing; (c) preference for complete freeze-drying or moderate freeze-drying in the case of entire embolizing; (d) the optimal ratio of e.g. protein/mucosaccharide/zinc, provided that a clinically required amount of a drug is entrapped in the composition; (e) preference or non-preference for chondroitin sulfate as mucosaccharide; and (f) possibility or impossibility of embolization using only a divalent metal ion according to properties of a drug depend on individual drugs.

### BRIEF DESCRIPTION OF THE DRAWINGS

FIG. 1 is a diagram showing the transition of the PC-BDNF blood level of a ddy mouse after administering a PC-BDNF-HAp preparation;
FIG. 2 is a view showing the transition of the IFN α blood level of a ddy mouse after administering an IFN α-HAp preparation;
FIG. 3 is a view showing the effect of the Zn concentration on the sustained release of an IFN-HAp preparation;
FIG. 4 is a view showing the difference in the combination of G-CSF with HAp between the case of being embolized and the case of being not embolized;
FIG. 5 is a view showing the results of in vitro elution of a drug; and
FIG. 6 is a view showing the comparison of *in vivo* solubility based on the difference of sintering temperatures.

### DETAILED DESCRIPTION OF THE PREFERRED EMBODIMENTS

Examples of the present invention will be described below.

### (Example 1)

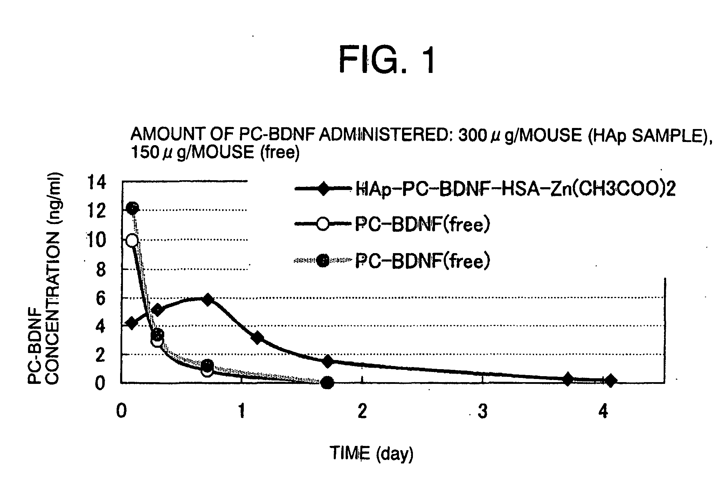

66 µl of a 4.54 mg/ml PC-BDNF (lecithinized BDNF) solution was added to 20 mg of porous hydroxyapatite microparticles (HAp) sintered at 180°C, and the mixture was stirred using a vortex mixer for one minute. 434 µl of a 0.1% HSA solution was added thereto, and the mixture was stirred again for one minute. The mixture was allowed to stand for three minutes, and then centrifuged at 1,000 rpm for three minutes to remove the supernatant liquid. 500 µl of a 5 mM Zn(CH₃COO)₂/5% mannitol solution was added to the deposit, and the mixture was stirred to prepare a sample. As a control, a sample in which 66 µl of a 4.54 mg/ml PC-BDNF solution was mixed with 434 µl of 5% mannitol was also prepared. 500 µl of each sample was subcutaneously administered to a six-week-old male ddy mouse. Two hours, seven hours, 17 hours, one day, two days, and four days after the administration, blood was collected from the orbit to determine the PC-BDNF blood level using an ELISA kit (Promega). The results indicated that an excellent sustained-release effect was obtained. The results are shown in FIG. 1.

### (Example 2)

0.854 ml of 225 µg/ml IFN α (interferon α, Sumitomo Pharmaceuticals) and 1.2 ml of 20 mg/ml HSA were mixed to prepare a protein-mixed solution. 0.856 ml of the protein-mixed solution was mixed with 200 mg of HAp sintered at 180°C, and the mixture was stirred to entrap a protein in HAp. 0.05 ml of 20 mg/ml chondroitin sulfate (CS, from Wako), 0.074 ml of H₂O, and 0.02 ml of 1 M Zn(CH₃COO)₂ were sequentially added thereto. The mixture was centrifuged at 15,000 rpm for five minutes. 2 ml of a 20 mM Zn(CH₃COO)₂/5% mannitol solution was added to the deposit to prepare a sample.

As a control, 0.644 ml of H₂O and 0.5 ml of 20% mannitol were added to 0.856 ml of an IFN α-containing protein-mixed solution to prepare an IFN α (free) solution.

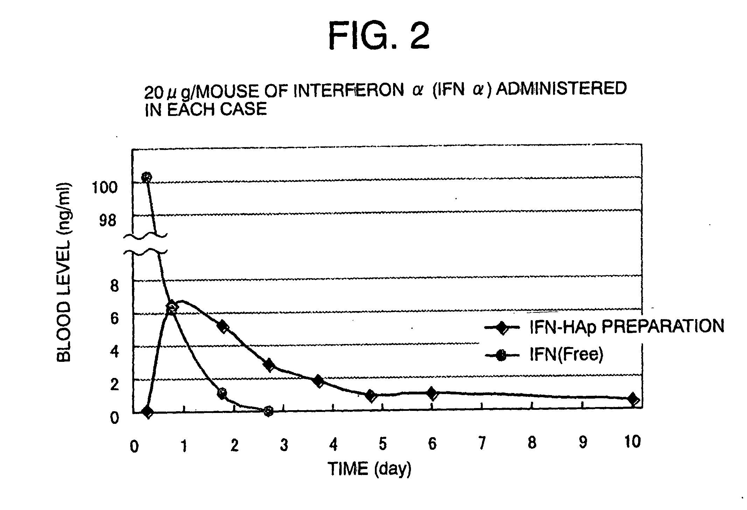

0.5 ml of the sample prepared above was subcutaneously administered to an eight-week-old male ddy mouse (weight: 33 to 40 g, SLC, Japan). Four hours and one to ten days after the administration, blood was collected from the orbit of the mouse. The IFN α concentration in this blood was determined using an ELISA kit (Biosource). The kinetics of the drug in blood are shown in FIG. 2. The results indicated that an excellent sustained-release effect was obtained.

### (Example 3)

0.06 ml of 0.937 mg/ml IFN α, 0.3 ml of 20 mg/ml HSA, 0.03 ml of 20 mg/ml CS, and 1.22 ml of H₂O were added to prepare a solution. 200 mg of HAp sintered at 180°C was added on this solution. A protein was entrapped in HAp by stirring, and then 10 ml of H₂O was added. The mixture was lightly stirred and centrifuged at 3,000 rpm for five minutes. The deposit was freeze-dried and divided in two. 1 ml of a 20 mM

Zn(CH₃COO)₂/5% mannitol solution was added to one division, while 1 ml of a 5 mM Zn(CH₃COO)₂/5% mannitol solution was added to the other division.

As a control, 0.015 ml of 0.937 mg/ml IFN α, 0.075 ml of 20 mg/ml HSA, 0.66 ml of H₂O, and 0.25 ml of 20% mannitol were added to prepare an IFN α (free) solution.

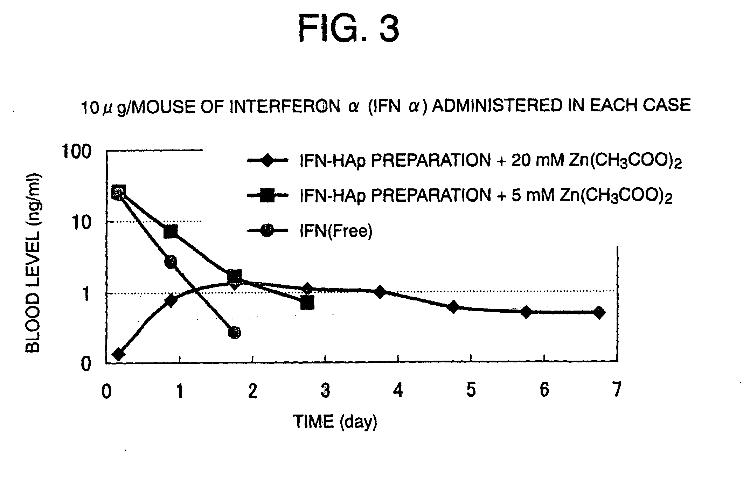

0.7 ml of the sample prepared above was subcutaneously administered to a seven-week-old male ddy mouse (weight: 31 to 33 g, SLC, Japan). Four hours and one to seven days after the administration, blood was collected from the orbit of the mouse. The IFN α concentration in this blood was determined using an ELISA kit (Biosource). The kinetics of the drug in blood are shown in FIG. 3. The results indicated that, when zinc was used 20 mM, the IFN α blood level was not sufficiently increased, but IFN α was released slowly for a long time. On the other hand, when zinc was used 5 mM, the IFN α blood level was sufficiently increased, but was reduced comparatively rapidly.

### (Example 4)

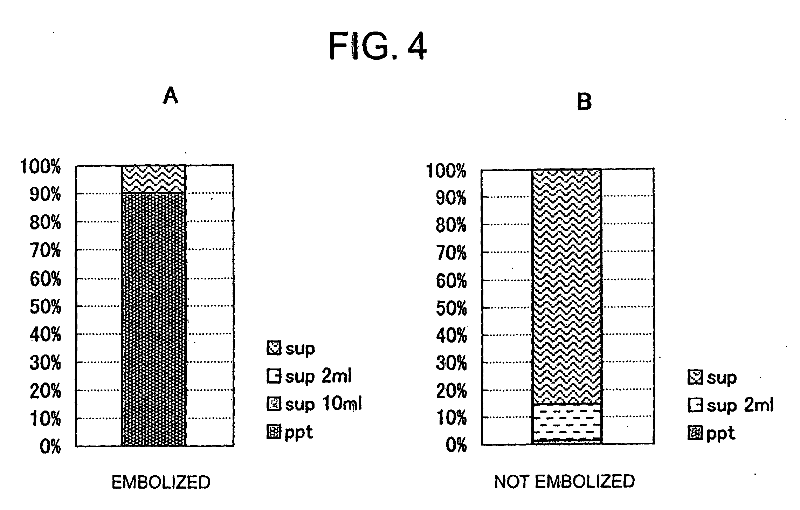

100 µl of a preliminarily mixed solution of G-CSF (3 µg/ml), HSA (30 µg/ml), and CaCl₂ (280 mg/ml) was added to 50 mg of HAp sintered at 180°C. The mixture was stirred using a vortex mixer for three minutes, allowed to stand for five minutes, and freeze-dried. 100 µl of a 220 mg/ml Na₂CO₃ solution was added to the resulting particles, and the mixture was stirred using a vortex mixer for three minutes. After further addition of 100 µl of water, the mixture was lightly stirred. A part of the mixture was collected for ELISA determination. The remaining part was centrifuged at 1,000 rpm for three minutes to collect the supernatant liquid. 2 ml of PBS was added to the deposit, and the mixture was lightly shaken at room temperature. After 0 hour and 0.5 hour, respectively, the supernatant liquid was collected. Further, 10 ml of PBS was added to the deposit, and the mixture was shaken at room temperature. After 0 hour and 0.5 hour, respectively, the supernatant liquid was collected. The final deposit and the supernatant liquids obtained on the way were subjected to determination using an ELISA kit (IBL). The final deposit was dissolved in 1% BSA/Tris-HCl (pH 5) and used for the ELISA determination. The results are shown in FIG. 4-A. 200 µl of a 1.5 µg/ml G-CSF solution was added to 50 mg of HAp to fill the pores, and 2 ml of PBS was added further. The same release test was conducted. The results are shown in FIG. 4-B. As shown in the figures, release was remarkably suppressed by the embolizing technology.

### (Example 5)

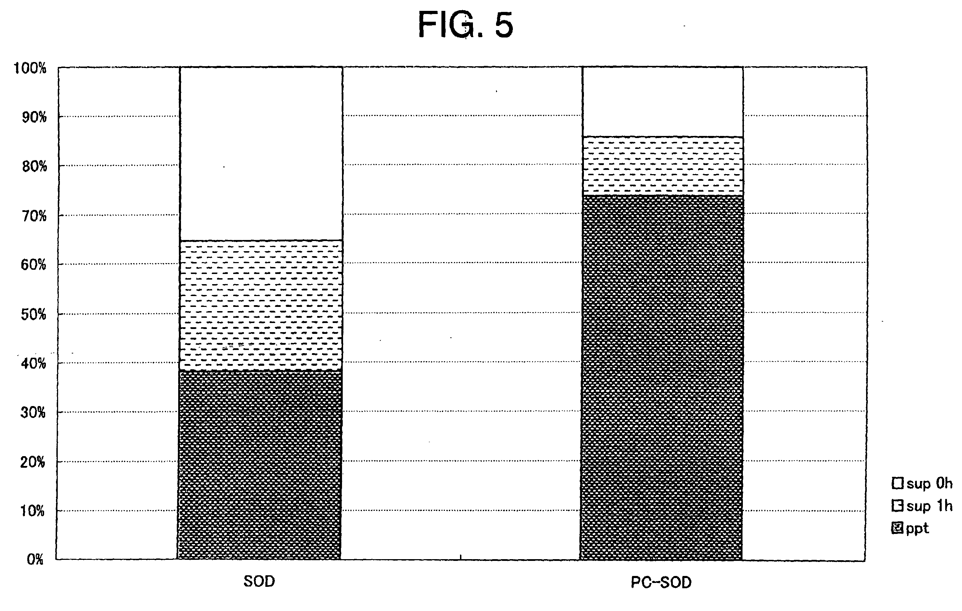

10 µl of a 100 mg/ml SOD solution or 25 µl of a 40 mg/ml PC-SOD (lecithinized SOD) solution was added to 50 mg of HAp sintered at 180°C, and the mixture was stirred using a vortex mixer for one minute. 990 µl or 975 µl of water was added thereto, and the mixture was stirred again for one minute. The mixture was allowed to stand for three minutes and then centrifuged at 1,000 rpm for three minutes to collect the supernatant liquid. 2 ml of water was added to the deposit, and the mixture was stirred and centrifuged at 1,000 rpm for three minutes to collect the supernatant liquid. Further, 2 ml of PBS was added to the deposit, and the mixture was stirred and centrifuged at 1,000 rpm for three minutes to collect the supernatant liquid. 1 ml of PBS was added to the resulting deposit, and the mixture was shaken at room temperature. After 0 hour and 1 hour, respectively, the supernatant liquid was collected. The supernatant liquids and the final deposit were subjected to determination using an BCA Assay (Pierce). The results are shown in FIG. 5. As shown in the figure, an increased amount of a protein was adsorbed on HAp by chemical modification.

### (Example 6)

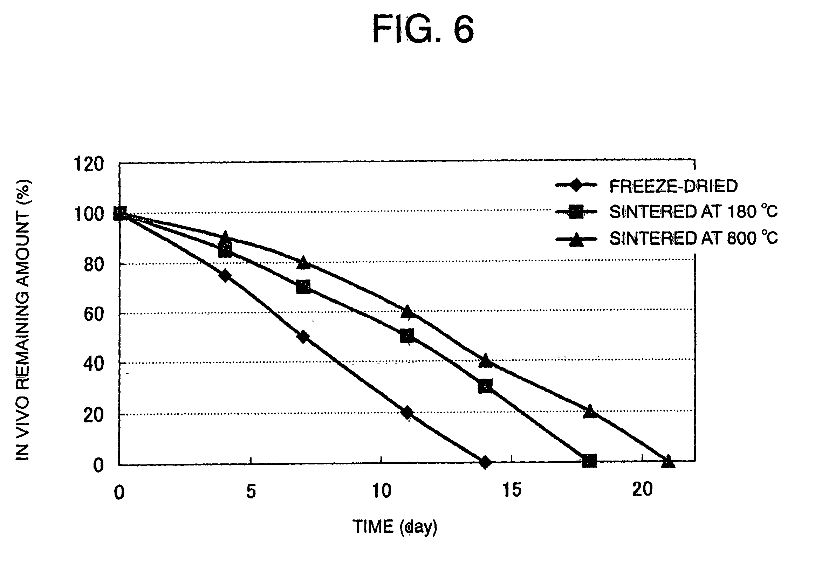

6 ml of 5% mannitol was added to 24 mg of freeze-dried HAp, 24 mg of HAp sintered at 180°C, and 24 mg of HAp sintered at 800°C, respectively. The mixtures were stirred using a vortex mixer for one minute to prepare HAp solutions. 500 µl each of these HAp solutions was administered to three places on the back of a thirteen-week-old male Wistar rat (weight: 330,400 g, SLC, Japan) so that the HAp solutions were not administered to the same place. After two hours and 4, 7, 11, 14, 18, and 21 days, these places on the back were dissected to take a photograph for examining the amount of remaining HAp. Later, several persons evaluated the approximate remaining amount of HAP visually using the photograph. The results indicated that HAp disappeared in two or three weeks in each case, but was more difficult to disappear as the sintering temperature was higher. The results are shown in FIG. 6.

### (Example 7)

Adsorption of G-CSF on hydroxyapatite using zinc

40 mg of hydroxyapatite particles were immersed in 100 µl of a G-CSF solution (100 µg/ml) for 10 minutes. Then, 900 µl of purified water was added, and the mixture was stirred and centrifuged. Then, the supernatant liquid was discarded. Purified water was added to the precipitate again, and the mixture was stirred and centrifuged to remove the excessive G-CSF. The precipitate was suspended in a pH 4 acetic acid buffer to elute G-CSF. After centrifugation, the amount of G-CSF in the supernatant liquid was determined by ELISA to determine the amount of G-CSF adsorbed. Almost no adsorption was observed (0.1 µg or less).

Then, the following procedure was conducted to attempt adsorption of zinc on hydroxyapatite particles and adsorption of G-CSF on the particles. 10 mg of hydroxyapatite was suspended in 200 µl of zinc acetate (5 mg/ml) . The suspension was allowed to stand for 10 minutes at room temperature, and then centrifuged at 10,000 rpm for 10 minutes to discard the supernatant liquid. The precipitate was suspended in 500 µl of purified water. The suspension was allowed to stand for 10 minutes at room temperature, and then centrifuged at 10,000 rpm for 10 minutes to discard the supernatant liquid. After the sediment was suspended and centrifuged again, the supernatant liquid was discarded. This precipitate was suspended in 0.5 ml of a 200 µg/ml or 1,000 µg/ml G-CSF solution. The suspension was allowed to stand for 10 minutes, and then centrifuged at 10,000 rpm for 10 minutes. The amount of G-CSF in the supernatant liquid was determined by an ELISA method. Further, G-CSF entrapped in hydroxyapatite was eluted from the precipitate using 0.1 M EDTA and a 1% HAS solution, and the eluate was centrifuged. Then, the G-CSF concentration in the supernatant liquid was determined by an ELISA method to determine the amount of G-CSF adsorbed on hydroxyapatite.

As shown in Table 1, when hydroxyapatite was preliminarily treated with zinc salt, 80.0 to 86.4% of G-CSF added to 10 mg of hydroxyapatite was adsorbed on the hydroxyapatite. 400 µg at maximum of G-CSF could be adsorbed on 10 mg of hydroxyapatite.

**Table 1:**

| Amount of G-CSF entrapped in hydroxyapatite (10 mg) bound with zinc | | | |
|---|---|---|---|
| Amount of hydroxyapatite (mg) | Total amount of G-CSF (µg) | Amount of free G-CSF (µg) | Amount of adsorbed G-CSF (µg) |
| 10 | 100 | 0.4 | 86.4 |
| 10 | 500 | 90.0 | 400.0 |

### (Example 8)

45 mg of porous hydroxyapatite (HAP) was accurately weighed. 30 µg of IFN from a 2.4 mg/ml interferon α (IFN) solution was added thereto, and the mixture was allowed to stand for 10 minutes. Then, 1 ml of a 20 mM/1 ml zinc acetate solution was added thereto, and the mixture was shaken for 30 minutes. This dispersion was washed by addition of 1.5 ml of water. When IFN in the washed solutionwas quantitatively determined, IFN was not detected. Specifically, all IFN was confirmed to be entrapped on HAP. In this manner, a microparticle preparation on which IFN as a protein was entrapped could be obtained without using an organic solvent. After the washing, 20 ml of a PBS solution containing FCS in an amount of 20% was added to the resulting powder, and the mixture was shaken at 37°C for 16 hours. IFN eluted in the supernatant liquid was quantitatively determined to calculate the percent elution. The results shown in Table 2 were obtained.

**Table 2:**

| Percent elution of IFN entrapped onto HAP | | |
|---|---|---|
| | | Eluted IFN (%) |
| HAP | Zinc acetate 0 mM | 92 |
| | Zinc acetate 20 mM | 87 |

Elution was suppressed by the addition of zinc acetate, and zinc acetate-added IFN was released more slowly for a long time as compared with zinc acetate-free IFN.

## Claims

1. A sustained-release composition **characterized in that** the composition comprises porous hydroxyapatite microparticles having pores charged by a biologically active drug, a human serum protein, and a mucopolysaccharide, and then embolized by adding a divalent metal ion.

2. The sustained-release composition according to claim 1, **characterized in that** the porous hydroxyapatite microparticles are obtained by spray-drying a suspension including hydroxyapatite and sintering the dried solid at a temperature from 100 to 800°C.

3. The sustained-release composition according to claim 1 or 2, **characterized in that** the porous hydroxyapatite microparticles have a particle size of 0.1 to 20 µm.

4. The sustained-release composition according to claim 1, **characterized in that** the biologically active drug is contained in the sustained-release composition in an amount of at least 0.01 wt%.

5. The sustained-release composition according to claim 1,
**characterized in that** the human serum protein is human serum albumin or γ-globulin.

6. The sustained-release composition according to claim 1 or 5, **characterized in that** the human serum protein is contained in the sustained-release composition in an amount of at least 1 wt%.

7. The sustained-release composition according to claim 1,
**characterized in that** the divalent metal ion is a zinc, calcium, or magnesium ion.

8. The sustained-release composition according to claim 1 or 6, **characterized in that** the divalent metal ion is contained in the sustained-release composition in an amount of at least 0.01 wt%.

9. The sustained-release composition according to claim 1, **characterized in that** the mucopolysaccharide is at least one selected from the group consisting of chondroitin sulfate, hyaluronic acid, heparin, heparan sulfate, dermatan sulfate, keratan sulfate, and the salts thereof.

10. The sustained-release composition according to claim 1 or 9, **characterized in that** the mucopolysaccharide is contained in the sustained-release composition in an amount of 1/100 or more of the human serum protein.

11. The sustained-release composition according to any one of claims 1 to 10, **characterized in that** the sustained-release composition is in a form suitable for subcutaneous injection, intradermal injection, intramuscular injection, intraocular administration, and dermal application.

12. A sustained-release preparation **characterized in that** the preparation comprises the composition according to claim 1 into which a pharmaceutically acceptable adjunct is optionally incorporated.

13. The preparation according to claim 12, **characterized in that** the pharmaceutically acceptable additive is a surfactant, preservative, or stabilizer.

14. A preparation **characterized in that** the preparation according to claim 12 is freeze-dried.

15. The preparation according to any one of claims 12 to 14, **characterized in that** the preparation is in a form suitable for subcutaneous injection, intradermal injection, intramuscular injection, intraocular administration, and dermal application.

16. A sustained-release composition **characterized in that** the composition comprises porous hydroxyapatite microparticles produced by which (1) the pores are charged by a biologically active drug, a human serum protein, and a mucopolysaccharide, (2) the microparticles are freeze-dried fully or moderately, and then (3) the resulting microparticles are embolized by adding a divalent metal ion solution.

17. A sustained-release composition **characterized in that** the composition comprises porous hydroxyapatite microparticles having pores charged by a biologically active drug and a human serum protein, and then embolized in the outer layer thereof by adding a divalent metal ion.

18. A sustained-release composition **characterized in that** the composition comprises porous hydroxyapatite microparticles having pores charged by a biologically active drug, a human serum albumin, and a water-soluble calcium salt one after another or at one time, and then embolized in the outer layer by adding sodium carbonate, sodium hydrogen carbonate, or an aqueous carbonate ion solution.

19. The sustained-release composition according to claim 18, **characterized in that** the water-soluble calcium salt is calcium chloride, calcium acetate, or calcium nitrate.

20. A sustained-release composition **characterized in that** the composition comprises porous hydroxyapatite microparticles produced by which (1) the pores are charged by a biologically active drug, a human serum albumin, and a water-soluble calcium salt one after another or at one time, (2) the microparticles are freeze-dried the pores fully or moderately, and then (3) the resulting microparticles are embolized by adding sodium carbonate, sodium hydrogen carbonate, or an aqueous carbonate ion solution.

21. A sustained-release composition **characterized in that** the composition comprises porous hydroxyapatite microparticles having pores charged by a biologically active drug and a water-soluble calcium salt one after another or at one time, and then embolized in the outer layer by adding sodium carbonate, sodium hydrogen carbonate, or an aqueous carbonate ion solution.

22. A sustained-release composition **characterized in that** the composition comprises porous hydroxyapatite microparticles having a biologically active drug highly binding to hydroxyapatite in the inner surface of pores.

23. A sustained-release composition **characterized in that** the composition comprises porous hydroxyapatite microparticles having pores binding the inner surface of pores in the microparticles with a biologically active drug, and further embolized by adding a divalent metal ion.

24. A sustained-release composition **characterized in that** the composition comprises porous hydroxyapatite microparticles having pores embolized by binding the inner surface of pores in the microparticles with a divalent metal ion, and further adding a biologically active drug.

25. The sustained-release composition according to claim 23 or 24, **characterized in that** the divalent metal ion is a zinc, copper, calcium, or magnesium ion.

26. A sustained-release composition for skin **characterized in that** the composition comprises porous hydroxyapatite microparticles filled with a dermatological therapeutic drug or a quasi-drug such as a sun screen together with a base, wherein the microparticles are mixed with an ointment, cream, or lotion.

27. A process for producing a sustained-release composition,
**characterized in that** the process comprises mixing porous hydroxyapatite microparticles with an aqueous solution comprising a biologically active drug and a human serum protein and stirring the mixture to prepare a suspension, mixing the suspension with an aqueous mucopolysaccharide solution and a divalent metal ion solution, and separating a resulting solid from the mixture.

28. A process for producing a sustained-release composition, **characterized in that** the process comprises mixing porous hydroxyapatite microparticles with an aqueous solution comprising a biologically active drug and a human serum protein and stirring the mixture to prepare a suspension, mixing the suspension with an aqueous mucopolysaccharide solution and a divalent metal ion solution, separating a resulting solid from the mixture, and further freeze-drying the solid.

29. A process for producing a sustained-release composition, **characterized in that** the process comprises mixing porous hydroxyapatite microparticles with an aqueous solution comprising a biologically active drug and a human serum protein and stirring the mixture to prepare a suspension, mixing the suspension with an aqueous mucopolysaccharide solution, separating a resulting solid from the mixture, freeze-drying the solid, adding a divalent metal ion solution to the solid, and further freeze-drying the mixture.

30. A process for producing a sustained-release composition, **characterized in that** the process comprises mixing porous hydroxyapatite microparticles with an aqueous solution comprising a biologically active drug, a human serum protein, and a water-soluble calcium salt and stirring the mixture to prepare a suspension, mixing the suspension with sodium carbonate, sodium hydrogen carbonate, or an aqueous carbonate ion solution, and separating a resulting solid from the mixture.

31. A process for producing a sustained-release composition,
**characterized in that** the process comprises mixing porous hydroxyapatite microparticles with an aqueous solution comprising a biologically active drug, a human serum protein, and a water-soluble calcium salt and stirring the mixture to prepare a suspension, separating a resulting solid from the suspension, freeze-drying the solid, adding sodium carbonate, sodium hydrogen carbonate, or an aqueous carbonate ion solution to the solid, and further freeze-drying the mixture.

32. A process for producing a sustained-release composition, **characterized in that** the process comprises mixing porous hydroxyapatite microparticles with an aqueous solution comprising a biologically active drug and a water-soluble calcium salt and stirring the mixture to prepare a suspension, separating a resulting solid from the suspension, freeze-drying the solid, adding sodium carbonate, sodium hydrogen carbonate, or an aqueous carbonate ion solution to the solid, and further freeze-drying the mixture.

33. A process for producing a sustained-release composition, **characterized in that** the process comprises mixing porous hydroxyapatite microparticles with an aqueous solution comprising a biologically active drug and stirring the mixture to prepare a suspension, mixing the suspension with a divalent metal ion solution, and separating a resulting solid from the mixture.

34. A process for producing a sustained-release composition, **characterized in that** the process comprises mixing porous hydroxyapatite microparticles with a divalent metal ion solution and stirring the mixture to prepare a suspension, mixing the suspension with an aqueous solution comprising a biologically active drug, and separating a resulting solid from the mixture.
